# EUROPEAN PATENT APPLICATION

(11) **EP 2 157 094 A1**
(43) Date of publication of application: **24.02.2010**
(21) Application number: 07817148.5
(22) Date of filing: 25.10.2007
(51) Int. Cl.: C07F 7/18, C07D 251/34, C07D 251/30, C08G 77/50, C08G 77/388

(54) **AN ISOCYANATE TRIMER HAVING THE SILANE OR FUNCTIONAL POLYSILOCANE STRUCTURE AND THEIR PREPARATION METHODS**

(30) Priority: 14.05.2007 CN 200710052179
(71) Applicant: Zhang, Qunchao, NO.1, Changjiangbumatou Yingcheng Hubei 432405 (CN); Wang, Decai, NO.1, Changjiangbumatou Yingcheng Hubei 432405 (CN)
(72) Inventor: Zhang, Qunchao, NO.1, Changjiangbumatou Yingcheng Hubei 432405 (CN); Wang, Decai, NO.1, Changjiangbumatou Yingcheng Hubei 432405 (CN)
(74) Representative: Weber, Jean-François
(86) International application number: PCT/CN2007/070957
(87) International publication number: WO 2008/138195

(57) **Abstract**

An isocyanate trimer having the silane structure or modified by the functional polysiloxane represented by the following formula 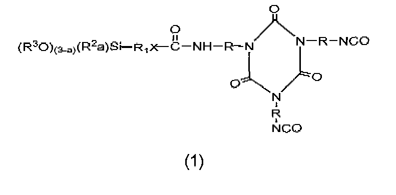 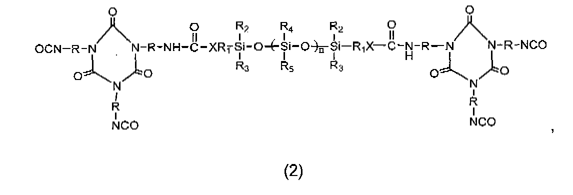 and their preparation methods, in which R is the group such as tolyl, ethylphenyl, 1,5-naphthyl, diphenylmethyl, hexamethyl, isophorone, 2,2,6-tirmethylcyclohexyl, 4,4'-biscyclohexylmethane which substituted by di-isocyanate; R₁ is the saturated or unsaturated, linear or branched alkyl, cyclic group, aryl etc., its main carbon atom is 1-8, and may be the homologue of aromatic hydrocarbon, R² and R³ each is C₁^{∼}C₆alkyl, aryl, trialkylsilanyl, methoxylalkoxyl, a is a positive integer from 0 to 3, R₁,R₂,R₃,R₄ or R₅ each is saturated or unsaturated, linear or branched alkyl, cycloalkyl, aryl, alkoxyl, cycloalkoxyl etc., which the amount of carbon atoms is 1^{∼}12, X mainly represents -NH-, -NHCH₂CH₂NH-, -NHCH₂CH₂NHCH₂CH₂NH-, -N-, -NHCONH-, , -S-, -O-, n is a positive integer which ≥0.

## Description

The invention relates to an isocyanate trimer modified with silane or functional polysiloxane and a method of preparing the same, and more particularly to an isocyanate trimer prepared by reacting active hydrogen-containing silane or active hydrogen-containing functional polysiloxanes with an isocyanate trimer as well as a preparation method thereof. The novel isocyanate trimer modified with silane or functional polysiloxane can be used in coatings, adhesives, sealants, elastomers, foams and so on, particularly for current production of quick-drying and highly decorative furniture and automobile, with high value. They can not only be used for preparation of polyurethanes, but also used for preparation of other polymers such as epoxy resins, polyacrylates, polyacrylamide, phenolic resin, etc.

Nowadays, silicone-modified polyurethane has aroused more and more attention in academy and industry. While maintaining the original excellent performance, silicone-modified polyurethane exhibits improved properties such as adhesion, wear resistance, chemical resistance, heat resistance, weather resistance, electrical insulation, fire retardancy, and hydrophobicity, and can be cured at room temperature. However, the above-mentioned silicone-modified polyurethane mainly focuses on the modification of an isocyanate prepolymer. There are few reports on silicone-modified isocyanate trimer.

The study on preparation and application of isocyanate trimers is very active at home and abroad. U. S. Pats. 2801244, 5264572, 0109665, 6515125, and 3996223 separately discloses a method of preparing an isocyanate trimer, all of which have different yield under different catalytic system. Isocyanate trimers have a high application value and are mainly used in coatings, adhesives, sealants, elastomers, foam plastics and so on, particularly for current production of quick-drying and highly decorative furniture and automobile. However, most isocyanate trimers are solid at room temperature, except that hexamethylene diisocyanate (HDI) trimer is liquid. Therefore, to develop an isocyanate trimer with high compatibility is of great significance.

The invention aims at providing an isocyanate trimer modified with silane and a method of preparing the same. The combination of saline with an isocyanate trimer can improve the compatibility of the isocyanate trimer. Polyurethane or other polymers (for example, epoxy resins, polymethacrylate, polyacrylamide, and phenolic resin) prepared by the modified isocyanate trimer can improve the heat resistance, weather resistance, electrical insulation, fire retardancy, and hydrophobicity.

So, the invention provides a modified isocyanate trimer modified with silane or functional polysiloxane and having formula of 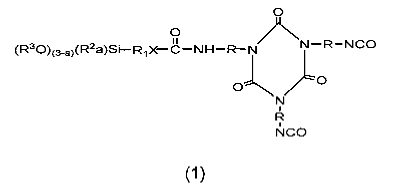 or

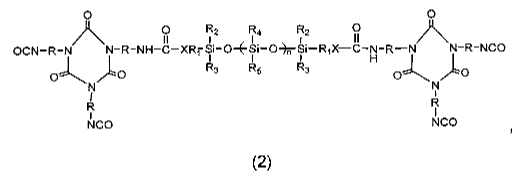

wherein

R represents tolyl, ethylphenyl, diphenylmethyl, 1,5-naphthyl, hexamethylene, isophorone, 2,2,6-trimethylcyclohexyl, 4,4-dicyclohexylmethyl;

R₁ is saturated or unsaturated and linear or branched C₁-₈ alkyl, cycloalkyl, aryl, or a homologue of aromatic hydrocarbon;

R² and R³ at each occurrence separately represents C₁-₆ alkyl, aryl, trialkylsilyl, or methoxyalkoxyl;

a is an integer from 0 to 3;

R₁, R₂, R₃, R₄, and R₅ at each occurrence separately represents saturated or unsaturated and linear or branched C₁-₁₂ alkyl, cycloalkyl, alkoxyl, aryl, or cycloalkoxyl;

X represents-NH-, -NHCH₂CH₂NH-, -NHCH₂CH₂NHCH₂CH₂NH-, -N-, -NHCONH-, 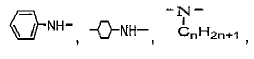 -S-, or -O-; and

n is an integer which is ≥ 0.

A method of preparing the modified isocyanate trimer represented by formula (1) or (2) comprises the steps of:

a) dissolving an isocyanate trimer into an inert solvent;

b) adding dropwise to a mixture of silane or functional polysiloxane and a catalyst;

c) stirring the resultant solution of b) at room temperature and in a dry environment; and

d) removing the inert solvent with reduced pressure distillation.

In this method, the isocyanate trimer has a formula of

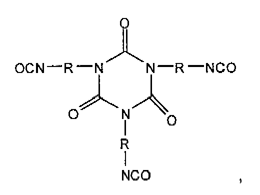

wherein R represents tolyl, ethylphenyl, diphenylmethyl, 1,5-naphthyl, hexamethylene, isophorone, 2,2,6-trimethylcyclohexyl, or 4,4-dicyclohexylmethyl.

In this method, the isocyanate is toluene diisocyanate (TDI), ethylbenzene diisocyanate (EDI), methylene diphenyl diisocyanate (MDI), 1,5-naphthalene diisocyanate (NDI), hexamethylene diisocyanate (HDI), isophorone diisocyanate (IPDI), 1,4-diisocyanate-2,2, 6-trimethyl cyclohexane (TMCDI), or 4,4-bis(isocyanatecyclohexyl)methane (HMDI).

In this method, the silane or functional polysiloxane has a formula of:

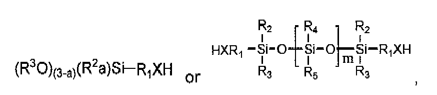

wherein R² and R³ at each occurrence separately represents C₁₋₆ alkyl, aryl, trialkylsilyl, or methoxylalkoxyl and a is an integer from 0 to 3; R₁, R₂, R₃. R₄, and R₅ at each occurrence separately represents saturated or unsaturated and linear or branched C₁₋₁₂ alkyl, aryl, cycloalkyl, alkoxyl, or cycloalkoxyl; X represents -NH-, -NHCH₂CH₂NH-, -NHCH₂CH₂NHCH₂CH₂NH-, -N-, -NHCONH-, 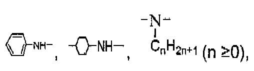 -S-, or -O-, and m is an integer from 0 to 30.

In this method, the catalyst is an organic tin compound including but not limited to dibutyltin dilaurate, dibutyltin maleate, dibutyltin diacetate, dibutyltin dilaurylmercaptan, and dimethyltin dichloride.

In this method, the amount of the catalyst is between 0.01 and 0.5 weight% of the total amount of the trimer and silicon compound.

In this method, the inert organic solvent which does not react with the reactants is hydrocarbon, chlorinated hydrocarbon, ester, or ether.

In this method, the hydrocarbon solvent is petroleum ether, linear or branched hydrocarbon with low boiling point, benzene, toluene, or xylene; the chlorinated hydrocarbon is chloroform, chlorobenzene, dichloromethane, or dichloroethane; ester is ethyl acetate; and ether is aether or tetrahydrofuran.

In this method, a molar ratio of the isocyanate trimer to the active hydrogen of silane or polysiloxane is 1:1.

Advantages of this invention are summarized below:

1. The isocyanate trimer modified with silanes or functional polysiloxanes has low viscosity, low volatile, low toxicity, high functionality, and high tolerance against xylene due to reduced isocyanate polarity, so that the compatibility of trimer with hydroxy resin has been improved significantly. In addition, heat resistance, weather resistance, and adhesion of isocyanate trimer have been further increased due to the introduction of organic silicon.

2. For the isocyanate trimer modified with silanes or functional polysiloxanes of the invention, isocyanate as a reactive functional group can react with polyester diols, polyether diols, hydroxy resins, and other polyols, and siloxane as another reactive functional group can react with inorganic materials or hydroxyl resins to form chemical bonds. In addition, the isocyanate trimer modified with silane or functional polysiloxane can be designed properly in accordance with the type of a polyol or polyol resin, which benefits the combination thereof.

3. For the reaction between isocyanate and active hydrogen of silanes or functional polysiloxanes to prepare carbamate, the introduction of an organometallic catalyst such as dibutyltin dilaurate can help obtain a high yield of product at low temperature (between -50°C and 30°C). Particularly, in case it is difficult for isocyanate trimer to react with active hydrogen of silanes or functional polysiloxanes due to space block, or the occurrence of a large amount of by-products at high temperature, the introduction of organic tin catalysts can smooth the reaction.

A monomer of the isocyanate trimer is selected from toluene diisocyanate (TDI), methylene diphenyl diisocyanate (MDI), hexamethylene diisocyanate (HDI), isophorone diisocyanate (IPDI), 1,4-diisocyanate-2,2,6-trimethyl cyclohexane (TMCDI), 4, 4-bis(isocyanatecyclohexyl) methane (HMDI), etc.

The silanes or functional polysiloxanes of the invention optionally have sulfhydryl, amino, diamino, triamino, secondary amino, hydroxy, hydroxyalkylamino, phenylamino, ureaalkyl, epoxy, N-alkyl amino, and N, N, N', N'-tetraalkyl guanidine with hydroxyl.

Particularly, a molar ratio of isocyanate trimer to the active hydrogen of silane or polysiloxane is 1:1.

In the reaction of isocyanate withsilane or functional polysiloxane, dibutyltin dilaurate, dibutyltin maleate, dibutyltin diacetate, dibutyltin dilaurylmercaptan, and dimethyltin dichloride is used as catalyst.

The reaction can be completed in a chlorinated alkyl solvent such as chloroform, cholorbenzene, dichloromethane, and dichloroethane, in an ether solvent such as aether, tetrahydrofuran, and cycloether, and in a hydrocarbon solvent such as petroleum ether, benzene, toluene, and xylene.

The isocyanate trimers of the invention have good compatibility with acrylic resin. As additives, the isocyanate trimers make acrylic resin with good gloss, scratching and wear resistance, water resistance, solvent resistance, chemical resistance, weather resistance, heat resistance, gloss retention, and good flexibility. In addition, it is also an important modifier of polyurethanes, epoxy resins, polyacrylamides, phenolic resins, etc.

For further illustrating the invention, experiments detailing a modified isocyanate trimer featuring high compatibility and a method of preparing the same are described below.

### Example 1

### Compound A

52.2 g of TDI trimer was dissolved in 400 mL of anhydrous tetrahydrofuran (THF), and the solution was added to a mixture of 22.1 g of γ-aminopropyl triethoxysilane and 0.07 g of dibutyltin dilaurate (DBTDL). The resultant solution was stirred for 5 hs at room temperature under a dry environment. Subsequently, THF was removed with reduced pressure distillation and 68.36 g of compound A was obtained, with a yield of 92%. The compound A has a formula of

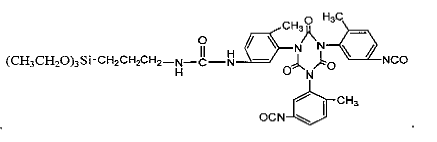

Elemental analysis of C₃₆H₄₁N₇O₉Si: measured value (calculated value) %: C 58.02(58.14), H 5.43(5.52), N 13.07(13.19), O 19.29(19.38), Si 3.68(3.77)¹HNMR (δ/ppm): 0.58(t, 2H), 1.22(q, 3H), 1.6(q, 2H), 2.0(m, 2H), 2.35(m, 3H), 2.65(q, 2H), 3.83(q, 2H), 6.9(t, 1H), 7.0(q, 1H), 7.26(m, 1H), 7.5(m, 2H), 7.9(s,1H).

### Example 2

### Compound B

52.2 g of TDI trimer was dissolved in 400 mL of anhydrous tetrahydrofuran (THF), and the solution was added to a mixture of 44 g of bis[y-(triethoxysilyl) propyl]amine and 0.07 g of dibutyltin dilaurate (DBTDL). The resultant solution was stirred for 5 hs at room temperature under a dry environment. Subsequently, THF was removed with reduced pressure distillation and 80.5 g of compound B was obtained, with a yield of 85%. The compound B has a formula of

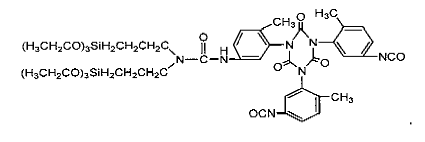

Elemental analysis of C₄₅H₆₁N₇O₁₂Si₂: measured value (calculated value)%: C 56.89(57.02), H 6.33(6.44), N 10.26(10.35), O 20.16(20.27), Si 5.79(5.91). ¹HNMR(δ/ppm): 0.58(t, 2H), 1.22(q, 3H), 1.6(q, 2H), 2.0(m, 2H), 2.35(m, 3H), 2.65(q, 2H), 3.83(q, 2H), 6.9(t, 1H), 7.0(q, 1H), 7.26(m, 1H), 7.5(m, 2H), 7.9(s,1H).

### Example 3

### Compound C

52.2 g of TDI trimer was dissolved in 400 mL of anhydrous tetrahydrofuran (THF), and the solution was added to a mixture of 22.2 g of γ-(β-amino-ethylamino) propyltrimethoxysilane and 0.07 g of dibutyltin dilaurate (DBTDL) The resultant solution was stirred for 5 hs at room temperature under a dry environment. Subsequently, THF was removed with reduced pressure distillation and 70.68 g of compound C was obtained, with a yield of 95%. The compound C has a formula of

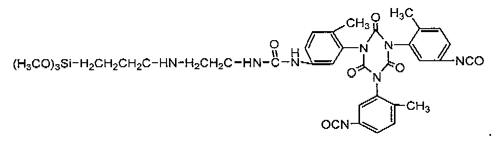

Elemental analysis of C₃₅H₄₀N₈O₉Si: measured value (calculated value)%: C 56.32(56.45), H 5.26(5.38), N 14.97(15.05), O 19-28(19.35), Si 3.69(3.76); ¹HNMR (δ/ppm): 1.22(q, 3H), 1.6(q, 2H), 2.0(m, 2H), 2.35(m, 3H), 2.65(q, 2H), 3.83(q, 2H), 6.0(s, 1H), 6.9(t,1H), 7.0(q, 1H), 7.26(m, 1H), 7.5(m, 2H), 7.9(s,1H).

### Example 4

### Compound D

52.2 g of TDI trimer was dissolved in 400 mL of anhydrous tetrahydrofuran (THF), and the solution was added to a mixture of 26.5 g of γ-(aminoethyl-aminoethylamino)propyltrimethoxysilane and 0.07 g of dibutyltin dilaurate (DBTDL). The resultant solution was stirred for 5 hs at room temperature under a dry environment. Subsequently, THF was removed with reduced pressure distillation and 75.5 g of compound D was obtained, with a yield of 96%. The compound D has a formula of

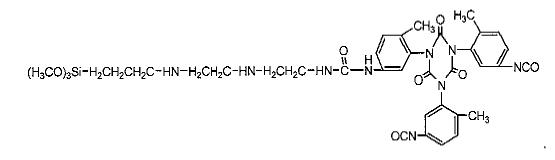

Elemental analysis of C₃₇H₄₅N₉O₉Si: measured value (calculated value)%: C 56.34(56.42), H 5.64(5.72), N 15.94(16.01), O 16.21(16.30), Si 3.42(3.56); ¹HNMR(δ/ppm): 0.58(q, 2H), 1.50(q, 2H), 2.0(q, 1H), 2.35(m, 1H), 2.55(q, 2H), 2.67(m, 2H), 2.81 (q, 2H), 3.28(q, 2H), 3.55(s, 3H), 6.0(s, 1H), 6.9(m, 1H), 7.0(q, 1H), 7.5(s, 1H), 7.9(m,1H).

### Example 5

### Compound E

52.2 g of TDI trimer was dissolved in 400 mL of anhydrous tetrahydrofuran (THF), and the solution was added to a mixture of 22.7 g of anilinomethyltrimethoxysilane and 0.08 g of dibutyltin dilaurate (DBTDL). The resultant solution was stirred for 8 hs at room temperature under a dry environment. Subsequently, THF was removed with reduced pressure distillation and 66.6 g of compound E was obtained, with a yield of 89%. The compound E has a formula of

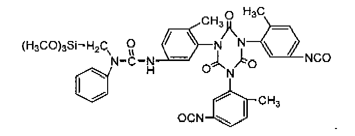

Elemental analysis of C₃₇H₃₅N₇O₉Si: measured value (calculated value) %: C 59.19(59.28), H 4.54(4.67), N 12.94(13.06), O 19.11(19.23), Si3.66(3.74); ¹HNMR(δ/ppm): 2.35(m,1H), 5(s, 2H, 2.4), 3.55(s, 3H), 6.0(s, 1H), 6.9(m, 1H), 7.0(q,1H), 7.4(q,1H), 7.5(s, 1H), 7.64(m,1H), 7.9(s,1H).

### Example 6

### Compound F

52.2 g of TDI trimer was dissolved in 400 mL of anhydrous tetrahydrofuran (THF), and the solution was added to a mixture of 21.3 g of p-aminophenyl- trimethoxysilane and 0.08 g of dibutyltin dilaurate (DBTDL). The resultant solution was stirred for 8 hs at room temperature under a dry environment. Subsequently, THF was removed with reduced pressure distillation and 63.0 g of compound F was obtained, with a yield of 86%. The compound F has a formula of

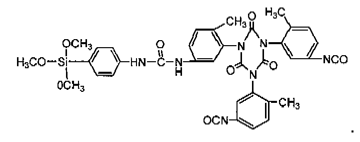

Elemental analysis of C₃₆H₃₃N₇O₈Si: measured value (calculated value)%: C 69.88(70.1), H 4.44(4.59), N 13.53(13.63), O 17.69(17.8), Si 3.76(3.89); ¹HNMR(δ/ppm): 0.66(m, 3H), 2.35(s,1H), 5(s, 3H, 3.5), 6.0(s, 1H), 6.9(q, 1H), 7.0(q,1H), 7.26(q,1H), 7.5(s, 1H), 7.6(m, 1H), 7.9(m,1H).

### Example 7

### Compound G

52.2 g of TDI trimer was dissolved in 400 mL of anhydrous tetrahydrofuran (THF), and the solution was added to a mixture of 23.5 g of n-butylamino-propyltrimethoxysilane and 0.07 g of dibutyltin dilaurate (DBTDL). The resultant solution was stirred for 6 hs at room temperature under a dry environment. Subsequently, THF was removed with reduced pressure distillation and 71.2 g of compound G was obtained, with a yield of 94%. The compound G has a formula of

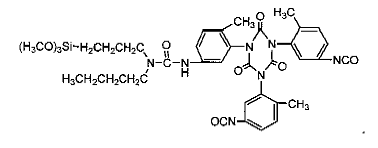

Elemental analysis of C₃₇H₄₃N₇O₉Si: measured value (calculated value)%: C 58.56(58.65), H 5.59(5.68), N 12.86(12.95), O 18.89(19.02), Si 3.62(3.70); ¹HNMR(δ/ppm): 0.96(q, 3H), 1.33(q,2H), 1.55(q,2H), 2.1(q, 2H), 2.35(m, 1H), 3.16(q, 2H), 3.55(s, 3H), 6.0(s, 1H), 6.9(q, 1H), 7.0(q, 1H), 7.26(q,1H), 7.5(s, 1H), 7.9(s,1H).

### Example 8

### Compound H

52.2 g of TDI trimer was dissolved in 400 mL of anhydrous tetrahydrofuran (THF), and the solution was added to a mixture of 22.2 g of y-ureido- propyltrimethoxysilane and 0.07 g of dibutyltin dilaurate (DBTDL). The resultant solution was stirred for 5 hs at room temperature under a dry environment. Subsequently, THF was removed with reduced pressure distillation and 70.7 g of compound H was obtained, with a yield of 95%. The compound H has a formula of

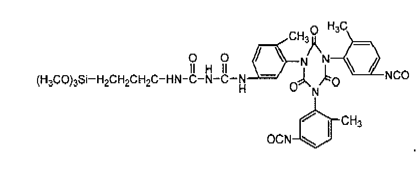

Elemental analysis of C₃₄H₃₆N₈O₁₀Si: measured value (calculated value) %: C 54.73(54.84), H 4.75(4.84), N 14.96(15.05), O 21.39(21.50), Si 3.63(3.76);¹HNMR(δ/ppm): 2.1(q, 2H), 2.35(m,1H), 3.55(m, 3H), 4.19(m, 2H), 6.0(s, 1H), 6.9(t, 1H), 7.0(t,1H), 7.26(t,1H), 7.5(s, 1H), 7.9(s,1H), 10(s, 1H).

### Example 9

### Compound I

52.2 g of TDI trimer was dissolved in 400 mL of anhydrous tetrahydrofuran (THF), and the solution was added to a mixture of 19.6 g of γ-mercapto- propyltrimethoxysilane and 0.07 g of dibutyltin dilaurate (DBTDL). The resultant solution was stirred for 8 hs at room temperature under a dry environment. Subsequently, THF was removed with reduced pressure distillation and 66.77 g of compound I was obtained, with a yield of 93%. The compound I has a formula of

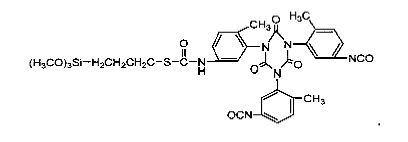

Elemental analysis of C₃₃H₃₄NsO₉SSi: measured value (calculated value)%: C 55.03(55.15), H 4.65(4.74), N 11.59(11.70), O 19.92(20.05), S 4.35(4.46), Si 3.79(3-90);1HNMR(δ/ppm): 2.35(m,1H), 2.8(q, 2H), 3.2(q, 2H),3.55(s, 1H), 6.9(t, 1H), 7.02(q,1H), 7.26(m,1H), 7.5(m, 1H), 7.9(s,1H), 8.0(s, 1H).

### Example 10

### Compound J

52.2 g of TDI trimer was dissolved in 400 mL of anhydrous tetrahydrofuran (THF), and the solution was added to a mixture of 18.0 g of γ-hydroxyl- propyltrimethoxysilane and 0.07 g of dibutyltin dilaurate (DBTDL). The resultant solution was stirred for 5 hs at room temperature under a dry environment. Subsequently, THF was removed with reduced pressure distillation and 65.98 g of compound J was obtained, with a yield of 94%. The compound J has a formula of

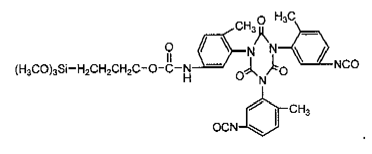

Elemental analysis of C₃₃H₃₄N₆O₁₀Si: measured value (calculated value)%: C 56.39(56.41), H 4.69(4.84), N 11.83(11.97), O 22.68(22.79), Si 3.89(3.99); ¹HNMR(δ/ppm): 0.58(t, 2H), 1.6(q, 2H), 2.35(m,1H), 3.55(s, 3H), 4.08(t, 2H), 6.9(q, 1H), 7.0(q,1H), 7.26(m,1H), 7.5(s, 1H), 7.9(s,1H), 8.0(s, 1H).

### Example 11

### Compound K

52.2 g of TDI trimer was dissolved in 400 mL of anhydrous tetrahydrofuran (THF), and the solution was added to a mixture of 35.1 g of N"-[2-hydroxy-3-[(3-trimethoxylsilyl) propoxyl]propyl]-N, N, N', N'-Tetra -methyl guanidine and 0.08 g of dibutyltin dilaurate (DBTDL). The resultant solution was stirred for 8 hs at room temperature under a dry environment. Subsequently, THF was removed with reduced pressure distillation and 78.57 g of compound K was obtained, with a yield of 90%. The compound K has a formula of

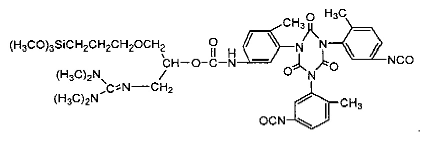

Elemental analysis of C₄₁H₅1N₉O₁₁Si: measured value (calculated value)%: C 65.72(65.75), H 5.74(5.84), N 14.33(14.43), O 20.03(20.16),Si 3.09(3.2); 1 HNMR (δ/ppm): 1.4(t, 2H), 1.57(q, 2H), 1.96(q, 2H), 2.35(s,1H), 2.47(s, 3H),3.55(s, 3H), 4.0(q, 2H), 5.0(m, 2H), 6.9(t, 1H), 7.0(q,1H), 7.5(m, 1H), 7.9(s,1H), 8.0(s, 1H).

### Example 12

### Compound L

104.4 g of TDI trimer was dissolved in 400 mL of anhydrous tetrahydrofuran (THF) and the solution was added to a mixture of 42 g of 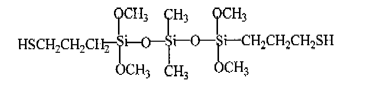 and 0.14 g of dibutyltin dilaurate (DBTDL). The resultant solution was stirred for 8 hs at room temperature under a dry environment. Subsequently, THF was removed with reduced pressure distillation and 135 g of compound L was obtained, with a yield of 92%. The compound L has a formula of

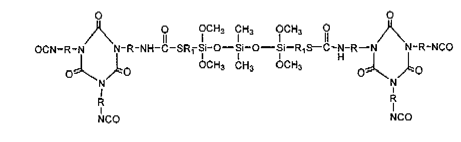

wherein R is 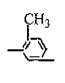 and R₁ is -CH₂CH₂CH₂-.

Elemental analysis of C₆₆H₅₀N₁₂O₁₈S₂Si₂: measured value (calculated value)%: C 55.76(55.85), H 3.44(3.53), N 11.74(11.85), O 20.22(20.31), S 4.42(4.51), Si 3.86(3.95);1HNMR(δ/ppm): 2.35(m,1H), 2.8(q, 2H), 3.2(q, 2H), 3.55(s, 1H), 6.9(t, 1H), 7.02(q,1H), 7.26(m, 1H),7.5(m, 1H), 7.9(s,1H), 8.0(s, 1H).

Other derivatives can be prepared by methods similar to the above mentioned. Some derivatives of the invention are listed in Tables 1 and 2.

In different compounds, the group represented by R has a formula of

| | | | |
|---|---|---|---|
| TDI₃ | 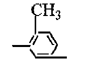 | MDI₃ | 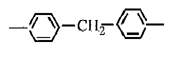 |
| NDI₃ | 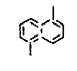 | HDI₃ | 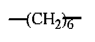 |
| IPDI₃ | 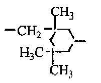 | TMCDI₃ | 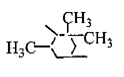 |
| HMDI₃ | 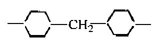 | | |

**Table 1 Derivatives represented by formula (1) of the invention**

| No. | R | X | R₁ | R² | R³ | a |
|---|---|---|---|---|---|---|
| 1 | TDI₃ | NH | CH₂CH₂CH₂ | CH₃ | CH₃O | 1 |
| 2 | TDI₃ | NH | CH₂CH₂CH₂ | CH₃ | CH₃O | 2 |
| 3 | TDI₃ | NHCH₂CH₂NH | CH₂CH₂CH₂ | CH₃ | CH₃O | 1 |
| 4 | TDI₃ | NHCH₂CH₂NH | CH₂CH₂CH₂ | CH₃ | CH₃O | 2 |
| 5 | TDI₃ | (C₆H₆)N | CH₂ | CH₃ | CH₃O | 1 |
| 6 | TDI₃ | (C₆H₆)N | CH₂ | CH₃ | CH₃CH₂O | 1 |
| 7 | TDI₃ | NHCH₂CH₂NHCH₂CH₂NH | CH₂CH₂CH₂ | CH₃ | CH₃O | 1 |
| 8 | TDI₃ | NHCH₂CH₂NHCH₂CH₂NH | CH₂CH₂CH₂ | CH₃ | CH₃O | 2 |
| 9 | TDI₃ | CH₃CH₂CH₂CH₂N | CH₂CH₂CH₂ | CH₃ | CH₃O | 1 |
| 10 | TDI₃ | CH₃CH₂CH₂CH₂N | CH₂CH₂CH₂ | CH₃ | CH₃O | 2 |
| 11 | TDI₃ | CH₃CH₂CH₂CH₂N | CH₂CH₂CH₂ | CH₃ | CH₃CH₂O | 1 |
| 12 | TDI₃ | NHCONH | CH₂CH₂CH₂ | CH₃ | CH₃O | 1 |
| 13 | TDI₃ | NHCONH | CH₂CH₂CH₂ | CH₃ | CH₃CH₂O | 1 |
| 14 | TDI₃ | NHCONHCH₂CH₂CH₂NH | CH₂CH₂CH₂ | CH₃ | CH₃O | 1 |
| 15 | TDI₃ | HS | CH₂CH₂CH₂ | CH₃ | CH₃O | 1 |
| 16 | TDI₃ | HS | CH₂CH₂CH₂ | CH₃ | CH₃O | 2 |
| 17 | TDI₃ | HS | CH₂CH₂CH₂ | CH₃ | CH₃CH₂O | 1 |
| 18 | TDI₃ | HO | CH₂CH₂CH₂ | CH₃ | CH₃O | 1 |
| 19 | TDI₃ | HO | CH₂CH₂CH₂ | CH₃ | CH₃CH₂O | 1 |
| 20 | TDI₃ | HO | CH₂cH₂cH₂ | CH₃ | CH₃O | 2 |
| 21 | MDI₃ | NH | CH₂CH₂CH₂ | CH₃ | CH₃O | 1 |
| 22 | MDI₃ | NH | CH₂CH₂CH₂ | CH₃ | CH₃O | 2 |
| 23 | MDI₃ | NHCH₂CH₂NH | CH₂CH₂CH₂ | CH₃ | CH₃O | 1 |
| 24 | MDI₃ | NHCH₂CH₂NH | CH₂CH₂CH₂ | CH₃ | CH₃O | 2 |
| 25 | MDI₃ | (C₆H₆)N | CH₂ | CH₃ | CH₃O | 1 |
| 26 | MDI₃ | (C₆H₆)N | CH₂ | CH₃ | CH₃CH₂O | 1 |
| 27 | MDI₃ | NHCH₂CH₂NHCH₂CH₂NH | CH₂CH₂CH₂ | CH₃ | CH₃O | 1 |
| 28 | MDI₃ | NHCH₂CH₂NHCH₂CH₂NH | CH₂CH₂CH₂ | CH₃ | CH₃O | 2 |
| 29 | MDI₃ | CH₃CH₂CH₂CH₂N | CH₂CH₂CH₂ | CH₃ | CH₃O | 1 |
| 30 | MDI₃ | CH₃CH₂CH₂CH₂N | CH₂CH₂CH₂ | CH₃ | CH₃O | 2 |
| 31 | MDI₃ | CH₃CH₂CH₂CH₂N | CH₂CH₂CH₂ | CH₃ | CH₃CH₂O | 1 |
| 32 | MDI₃ | NHCONH | CH₂CH₂CH₂ | CH₃ | CH₃O | 1 |
| 33 | MDI₃ | NHCONH | CH₂CH₂CH₂ | CH₃ | CH₃CH₂O | 1 |
| 34 | MDI₃ | NHCONHCH₂CH₂CH₂NH | CH₂CH₂CH₂ | CH₃ | CH₃O | 1 |
| 35 | MDI₃ | HS | CH₂CH₂CH₂ | CH₃ | CH₃O | 1 |
| 36 | MDI₃ | HS | CH₂CH₂CH₂ | CH₃ | CH₃O | 2 |
| 37 | MDI₃ | HS | CH₂CH₂CH₂ | CH₃ | CH₃CH₂O | 1 |
| 38 | MDI₃ | HO | CH₂CH₂CH₂ | CH₃ | CH₃O | 1 |
| 39 | MDI₃ | HO | CH₂CH₂CH₂ | CH₃ | CH₃CH₂O | 1 |
| 40 | MDI₃ | HO | CH₂CH₂CH₂ | CH₃ | CH₃O | 2 |
| 41 | NDI₃ | NH | CH₂CH₂CH₂ | CH₃ | CH₃O | 1 |
| 42 | NDI₃ | NH | CH₂CH₂CH₂ | CH₃ | CH₃O | 2 |
| 43 | NDI₃ | NHCH₂CH₂NH | CH₂CH₂CH₂ | CH₃ | CH₃O | 1 |
| 44 | NDI₃ | NHCH₂CH₂NH | CH₂CH₂CH₂ | CH₃ | CH₃O | 2 |
| 45 | NDI₃ | (C₆H₆)N | CH₂ | CH₃ | CH₃O | 1 |
| 46 | NDI₃ | (C₆H₆)N | CH₂ | CH₃ | CH₃CH₂O | 1 |
| 47 | NDI₃ | NHCH₂CH₂NHCH₂CH₂NH | CH₂CH₂CH₂ | CH₃ | CH₃O | 1 |
| 48 | NDI₃ | NHCH₂CH₂NHCH₂CH₂NH | CH₂CH₂CH₂ | CH₃ | CH₃O | 2 |
| 49 | NDI₃ | CH₃CH₂CH₂CH₂N | CH₂CH₂CH₂ | CH₃ | CH₃O | 1 |
| 50 | NDI₃ | CH₃CH₂CH₂CH₂N | CH₂CH₂CH₂ | CH₃ | CH₃O | 2 |
| 51 | NDI₃ | CH₃CH₂CH₂CH₂N | CH₂CH₂CH₂ | CH₃ | CH₃CH₂O | 1 |
| 52 | NDI₃ | NHCONH | CH₂CH₂CH₂ | CH₃ | CH₃O | 1 |
| 53 | NDI₃ | NHCONH | CH₂CH₂CH₂ | CH₃ | CH₃CH₂O | 1 |
| 54 | NDI₃ | NHCONHCH₂CH₂CH₂NH | CH₂CH₂CH₂ | CH₃ | CH₃O | 1 |
| 55 | NDI₃ | HS | CH₂CH₂CH₂ | CH₃ | CH₃O | 1 |
| 56 | NDI₃ | HS | CH₂CH₂CH₂ | CH₃ | CH₃O | 2 |
| 57 | NDI₃ | HS | CH₂CH₂CH₂ | CH₃ | CH₃CH₂O | 1 |
| 58 | NDI₃ | HO | CH₂CH₂CH₂ | CH₃ | CH₃O | 1 |
| 59 | NDI₃ | HO | CH₂CH₂CH₂ | CH₃ | CH₃CH₂O | 1 |
| 60 | NDI₃ | HO | CH₂CH₂CH₂ | CH₃ | CH₃O | 2 |
| 61 | HDI₃ | NH | CH₂CH₂CH₂ | CH₃ | CH₃O | 1 |
| 62 | HDI₃ | NH | CH₂CH₂CH₂ | CH₃ | CH₃O | 2 |
| 63 | HDI₃ | NHCH₂CH₂NH | CH₂CH₂CH₂ | CH₃ | CH₃O | 1 |
| 64 | HDI₃ | NHCH₂CH₂NH | CH₂CH₂CH₂ | CH₃ | CH₃O | 2 |
| 65 | HDI₃ | (C₆H₆)N | CH₂ | CH₃ | CH₃O | 1 |
| 66 | HDI₃ | (C₆H₆)N | CH₂ | CH₃ | CH₃CH₂O | 1 |
| 67 | HDI₃ | NHCH₂CH₂NHCH₂CH₂NH | CH₂CH₂CH₂ | CH₃ | CH₃O | 1 |
| 68 | HDI₃ | NHCH₂CH₂NHCH₂CH₂NH | CH₂CH₂CH₂ | CH₃ | CH₃O | 2 |
| 69 | HDI₃ | CH₃CH₂CH₂CH₂N | CH₂CH₂CH₂ | CH₃ | CH₃O | 1 |
| 70 | HDI₃ | CH₃CH₂CH₂CH₂N | CH₂CH₂CH₂ | CH₃ | CH₃O | 2 |
| 71 | HDI₃ | CH₃CH₂CH₂CH₂N | CH₂CH₂CH₂ | CH₃ | CH₃CH₂O | 1 |
| 72 | HDI₃ | NHCONH | CH₂CH₂CH₂ | CH₃ | CH₃O | 1 |
| 73 | HDI₃ | NHCONH | CH₂CH₂CH₂ | CH₃ | CH₃CH₂O | 1 |
| 74 | HDI₃ | NHCONHCH₂CH₂CH₂NH | CH₂CH₂CH₂ | CH₃ | CH₃O | 1 |
| 75 | HDI₃ | HS | CH₂CH₂CH₂ | CH₃ | CH₃O | 1 |
| 76 | HDI₃ | HS | CH₂CH₂CH₂ | CH₃ | CH₃O | 2 |
| 77 | HDI₃ | HS | CH₂CH₂CH₂ | CH₃ | CH₃CH₂O | 1 |
| 78 | HDI₃ | HO | CH₂CH₂CH₂ | CH₃ | CH₃O | 1 |
| 79 | HDI₃ | HO | CH₂CH₂CH₂ | CH₃ | CH₃CH₂O | 1 |
| 80 | HDI₃ | HO | CH₂CH₂CH₂ | CH₃ | CH₃O | 2 |
| 81 | IPDI₃ | NH | CH₂CH₂CH₂ | CH₃ | CH₃O | 1 |
| 82 | IPDI₃ | NH | CH₂CH₂CH₂ | CH₃ | CH₃O | 2 |
| 83 | IPDI₃ | NHCH₂CH₂NH | CH₂CH₂CH₂ | CH₃ | CH₃O | 1 |
| 84 | IPDI₃ | NHCH₂CH₂NH | CH₂CH₂CH₂ | CH₃ | CH₃O | 2 |
| 85 | IPDI₃ | (C₆H₆)N | CH₂ | CH₃ | CH₃O | 1 |
| 86 | IPDI₃ | (C₆H₆)N | CH₂ | CH₃ | CH₃CH₂O | 1 |
| 87 | IPDI₃ | NHCH₂CH₂NHCH₂CH₂NH | CH₂CH₂CH₂ | CH₃ | CH₃O | 1 |
| 88 | IPDI₃ | NHCH₂CH₂NHCH₂CH₂NH | CH₂CH₂CH₂ | CH₃ | CH₃O | 2 |
| 89 | IPDI₃ | CH₃CH₂CH₂CH₂N | CH₂CH₂CH₂ | CH₃ | CH₃O | 1 |
| 90 | IPDI₃ | CH₃CH₂CH₂CH₂N | CH₂CH₂CH₂ | CH₃ | CH₃O | 2 |
| 91 | IPDI₃ | CH₃CH₂CH₂CH₂N | CH₂CH₂CH₂ | CH₃ | | 1 |
| 92 | IPDI₃ | NHCONH | CH₂CH₂CH₂ | CH₃ | CH₃O | 1 |
| 93 | IPDI₃ | NHCONH | CH₂CH₂CH₂ | CH₃ | | 1 |
| 94 | IPDI₃ | NHCONHCH₂CH₂CH₂NH | CH₂CH₂CH₂ | CH₃ | CH₃O | 1 |
| 95 | IPDI₃ | HS | CH₂CH₂CH₂ | CH₃ | CH₃O | 1 |
| 96 | IPDI₃ | HS | CH₂CH₂CH₂ | CH₃ | CH₃O | 2 |
| 97 | IPDI₃ | HS | CH₂CH₂CH₂ | CH₃ | CH₃CH₂O | 1 |
| 98 | IPDI₃ | HO | CH₂CH₂CH₂ | CH₃ | CH₃O | 1 |
| 99 | IPDI₃ | HO | CH₂CH₂CH₂ | CH₃ | CH₃CH₂O | 1 |
| 100 | IPDI₃ | HO | CH₂CH₂CH₂ | CH₃ | CH₃O | 2 |
| 101 | TMCDI₃ | NH | CH₂CH₂CH₂ | CH₃ | CH₃O | 1 |
| 102 | TMCDI₃ | NH | CH₂CH₂CH₂ | CH₃ | CH₃O | 2 |
| 103 | TMCDI₃ | NHCH₂CH₂NH | CH₂CH₂CH₂ | CH₃ | CH₃O | 1 |
| 104 | TMCDI₃ | NHCH₂CH₂NH | CH₂CH₂CH₂ | CH₃ | CH₃O | 2 |
| 105 | TMCDI₃ | (C₆H₆)N | CH₂ | CH₃ | CH₃O | 1 |
| 106 | TMCDI₃ | (C₆H₆)N | CH₂ | CH₃ | CH₃CH₂O | 1 |
| 107 | TMCDI₃ | NHCH₂CH₂NHCH₂CH₂NH | CH₂CH₂CH₂ | CH₃ | CH₃O | 1 |
| 108 | TMCDI₃ | NHCH₂CH₂NHCH₂CH₂NH | CH₂CH₂CH₂ | CH₃ | CH₃O | 2 |
| 109 | TMCDI₃ | CH₃CH₂CH₂CH₂N | CH₂CH₂CH₂ | CH₃ | CH₃O | 1 |
| 110 | TMCDI₃ | CH₃CH₂CH₂CH₂N | CH₂CH₂CH₂ | CH₃ | CH₃O | 2 |
| 111 | TMCDI₃ | CH₃CH₂CH₂CH₂N | CH₂CH₂CH₂ | CH₃ | CH₃CH₂O | 1 |
| 112 | TMCDI₃ | NHCONH | CH₂CH₂CH₂ | CH₃ | CH₃O | 1 |
| 113 | TMCDI₃ | NHCONH | CH₂CH₂CH₂ | CH₃ | CH₃CH₂O | 1 |
| 114 | TMCDI₃ | NHCONHCH₂CH₂CH₂NH | CH₂CH₂CH₂ | CH₃ | CH₃O | 1 |
| 115 | TMCDI₃ | HS | CH₂CH₂CH₂ | CH₃ | CH₃O | 1 |
| 116 | TMCDI₃ | HS | CH₂CH₂CH₂ | CH₃ | CH₃O | 2 |
| 117 | TMCDI₃ | HS | CH₂CH₂CH₂ | CH₃ | CH₃CH₂O | 1 |
| 118 | TMCDI₃ | HO | CH₂CH₂CH₂ | CH₃ | CH₃O | 1 |
| 119 | TMCDI₃ | HO | CH₂CH₂CH₂ | CH₃ | CH₃CH₂O | 1 |
| 120 | TMCDI₃ | HO | CH₂CH₂CH₂ | CH₃ | CH₃O | 2 |
| 121 | HMDI₃ | NH | CH₂CH₂CH₂ | CH₃ | CH₃O | 1 |
| 122 | WADI3 | NH | CH₂CH₂CH₂ | CH₃ | CH₃O | 2 |
| 123 | HMDI₃ | NHCH₂CH₂NH | CH₂CH₂CH₂ | CH₃ | CH₃O | 1 |
| 124 | HMDI₃ | NHCH₂CH₂NH | CH₂CH₂CH₂ | CH₃ | CH₃O | 2 |
| 125 | HMDI₃ | (C₆H₆)N | CH₂ | CH₃ | CH₃O | 1 |
| 126 | HMDI₃ | (C₆H₆)N | CH₂ | CH₃ | CH₃CH₂O | 1 |
| 127 | HMDI₃ | NHCH₂CH₂NHCH₂CH₂NH | CH₂CH₂CH₂ | CH₃ | CH₃O | 1 |
| 128 | HMDI₃ | NHCH₂CH₂NHCH₂CH₂NH | CH₂CH₂CH₂ | CH₃ | CH₃O | 2 |
| 129 | HMDI₃ | CH₃CH₂CH₂CH₂N | CH₂CH₂CH₂ | CH₃ | CH₃O | 1 |
| 130 | HMDI₃ | CH₃CH₂CH₂CH₂N | CH₂CH₂CH₂ | CH₃ | CH₃O | 2 |
| 131 | HMDI₃ | CH₃CH₂CH₂CH₂N | CH₂CH₂CH₂ | CH₃ | CH₃CH₂O | 1 |
| 132 | HMDI₃ | NHCONH | CH₂CH₂CH₂ | CH₃ | CH₃O | 1 |
| 133 | HMDI₃ | NHCONH | CH₂CH₂CH₂ | CH₃ | CH₃CH₂O | 1 |
| 134 | HMDI₃ | NHCONHCH₂CH₂CH₂NH | CH₂CH₂CH₂ | H3 | CH₃O | 1 |
| 135 | HMDI₃ | HS | CH₂CH₂CH₂ | CH₃ | CH₃O | 1 |
| 136 | HMDI₃ | HS | CH₂CH₂CH₂ | CH₃ | CH₃O | 2 |
| 137 | HMDI₃ | HS | CH₂CH₂CH₂ | CH₃ | CH₃CH₂O | 1 |
| 138 | HMDI₃ | HO | CH₂CH₂CH₂ | CH₃ | CH₃O | 1 |
| 139 | HMDI₃ | HO | CH₂CH₂CH₂ | CH₃ | CH₃CH₂O | 1 |
| 140 | HMDI₃ | HO | CH₂CH₂CH₂ | CH₃ | CH₃O | 2 |

**Table 2 Derivatives represented by formula (2) of the invention**

| No. | R | X | R₁ | R₂=R₄ | R₃=R₅ | m |
|---|---|---|---|---|---|---|
| 141 | TDI₃ | NH | CH₂CH₂CH₂ | CH₃ | CH₃O | 1-10 |
| 142 | TDI₃ | NH | CH₂CH₂CH₂ | CH₃O | CH₃ | 10-20 |
| 143 | TDI₃ | NH | CH₂CH₂CH₂ | CH₃ | CH₃O | 20-30 |
| 144 | TDI₃ | NHCH₂CH₂NH | CH₂CH₂CH₂ | CH₃ | CH₃O | 1-10 |
| 145 | TDI₃ | NHCH₂CH₂NH | CH₂CH₂CH₂ | CH₃O | CH₃ | 10-20 |
| 146 | TDI₃ | NHCH₂CH₂NH | CH₂CH₂CH₂ | CH₃ | CH₃O | 20-30 |
| 147 | TDI₃ | (C₆H₆)N | CH₂ | CH₃ | CH₃O | 1-10 |
| 148 | TDI₃ | (C₆H₆)N | CH₂ | CH₃O | CH₃ | 10-20 |
| 149 | TDI₃ | (C₆H₆)N | CH₂ | CH₃ | CH₃O | 20-30 |
| 150 | TDI₃ | NHCH₂CH₂NHCH₂CH₂NH | CH₂CH₂CH₂ | CH₃ | CH₃O | 1-10 |
| 151 | TDI₃ | NHCH₂CH₂NHCH₂CH₂NH | CH₂CH₂CH₂ | CH₃O | CH₃ | 10-20 |
| 152 | TDI₃ | NHCH₂CH₂NHCH₂CH₂NH | CH₂CH₂CH₂ | CH₃ | CH₃O | 20-30 |
| 153 | TDI₃ | CH₃CH₂CH₂CH₂N | CH₂CH₂CH₂ | CH₃ | CH₃O | 1-10 |
| 154 | TDI₃ | CH₃CH₂CH₂CH₂N | CH₂CH₂CH₂ | CH₃O | CH₃ | 10-20 |
| 155 | TDI₃ | CH₃CH₂CH₂CH₂N | CH₂CH₂CH₂ | CH₃ | CH₃O | 20-30 |
| 156 | TDI₃ | NHCONH | CH₂CH₂CH₂ | CH₃ | CH₃O | 1-10 |
| 157 | TDI₃ | NHCONH | CH₂CH₂CH₂ | CH₃O | CH₃ | 10-20 |
| 158 | TDI₃ | NHCONH | CH₂CH₂CH₂ | CH₃ | CH₃O | 20-30 |
| 159 | TDI₃ | HS | CH₂CH₂CH₂ | CH₃ | CH₃O | 1-10 |
| 160 | TDI₃ | HS | CH₂CH₂CH₂ | CH₃O | CH₃ | 10-20 |
| 161 | TDI₃ | HS | CH₂CH₂CH₂ | CH₃ | CH₃O | 20-30 |
| 162 | TDI₃ | HO | CH₂CH₂CH₂ | CH₃ | CH₃O | 1-10 |
| 163 | TDI₃ | HO | CH₂CH₂CH₂ | CH₃O | CH₃ | 10-20 |
| 164 | TDI₃ | HO | CH₂CH₂CH₂ | CH₃ | CH₃O | 20-30 |
| 165 | MDI₃ | NH | CH₂CH₂CH₂ | CH₃ | CH₃O | 1-10 |
| 166 | MDI₃ | NH | CH₂CH₂CH₂ | CH₃O | CH₃ | 10-20 |
| 167 | MDI₃ | NH | CH₂CH₂CH₂ | CH₃ | CH₃O | 20-30 |
| 168 | MDI₃ | NHCH₂CH₂NH | CH₂CH₂CH₂ | CH₃O | CH₃ | 1-10 |
| 169 | MDI₃ | NHCH₂CH₂NH | CH₂CH₂CH₂ | CH₃ | CH₃O | 10-20 |
| 170 | MDI₃ | NHCH₂CH₂NH | CH₂CH₂CH₂ | CH₃O | CH₃ | 20-30 |
| 171 | MDI₃ | (C₆H₆)N | CH₂ | CH₃ | CH₃O | 1-10 |
| 172 | MDI₃ | (C₆H₆)N | CH₂ | CH₃O | CH₃ | 10-20 |
| 173 | MDI₃ | (C₆H₆)N | CH₂ | CH₃ | CH₃O | 20-30 |
| 174 | MDI₃ | NHCH₂CH₂NHCH₂CH₂NH | CH₂CH₂CH₂ | CH₃ | CH₃O | 1-10 |
| 175 | MDI₃ | NHCH₂CH₂NHCH₂CH₂NH | CH₂CH₂CH₂ | CH₃O | CH₃ | 10-20 |
| 176 | MDI₃ | NHCH₂CH₂NHCH₂CH₂NH | CH₂CH₂CH₂ | CH₃ | CH₃O | 20-30 |
| 177 | MDI₃ | CH₃CH₂CH₂CH₂N | CH₂CH₂CH₂ | CH₃O | CH₃ | 1-10 |
| 178 | MDI₃ | CH₃CH₂CH₂CH₂N | CH₂CH₂CH₂ | CH₃ | CH₃O | 10-20 |
| 179 | MDI₃ | CH₃CH₂CH₂CH₂N | CH₂CH₂CH₂ | CH₃O | CH₃ | 20-30 |
| 180 | MDI₃ | NHCONH | CH₂CH₂CH₂ | CH₃ | CH₃O | 1-10 |
| 181 | MDI₃ | NHCONH | CH₂CH₂CH₂ | CH₃O | CH₃ | 10-20 |
| 182 | MDI₃ | NHCONH | CH₂CH₂CH₂ | CH₃ | CH₃O | 20-30 |
| 183 | MDI₃ | HS | CH₂CH₂CH₂ | CH₃O | CH₃ | 1-10 |
| 184 | MDI₃ | HS | CH₂CH₂CH₂ | CH₃ | CH₃O | 10-20 |
| 185 | MDI₃ | HS | CH₂CH₂CH₂ | CH₃O | CH₃ | 20-30 |
| 186 | MDI₃ | HO | CH₂CH₂CH₂ | CH₃ | CH₃O | 1-10 |
| 187 | MDI₃ | HO | CH₂CH₂CH₂ | CH₃O | CH₃ | 10-20 |
| 188 | MDI₃ | HO | CH₂CH₂CH₂ | CH₃ | CH₃O | 20-30 |
| 189 | NDI₃ | NH | CH₂CH₂CH₂ | CH₃ | CH₃O | 1-10 |
| 190 | NDI₃ | NH | CH₂CH₂CH₂ | CH₃O | CH₃ | 10-20 |
| 191 | NDI₃ | NH | CH₂CH₂CH₂ | CH₃ | CH₃O | 20-30 |
| 192 | NDI₃ | NHCH₂CH₂NH | CH₂CH₂CH₂ | CH₃O | CH₃ | 1-10 |
| 193 | NDI₃ | NHCH₂CH₂NH | CH₂CH₂CH₂ | CH₃ | CH₃O | 10-20 |
| 194 | NDI₃ | NHCH₂CH₂NH | CH₂CH₂CH₂ | CH₃O | CH₃ | 20-30 |
| 195 | NDI₃ | (C₆H₆)N | CH₂ | CH₃ | CH₃O | 1-10 |
| 196 | NDI₃ | (C₆H₆)N | CH₂ | CH₃O | CH₃ | 10-20 |
| 197 | NDI₃ | (C₆H₆)N | CH₂ CH₂ | CH₃ | CH₃O | 20-30 |
| 198 | NDI₃ | NHCH₂CH₂NHCH₂CH₂NH | CH₂CH₂CH₂ | CH₃O | CH₃ | 1-10 |
| 199 | NDI₃ | NHCH₂CH₂NHCH₂CH₂NH | CH₂CH₂CH₂ | CH₃ | CH₃O | 10-20 |
| 200 | NDI₃ | NHCH₂CH2NHCH₂CH₂NH | CH₂CH₂CH₂ | CH₃O | CH₃ | 20-30 |
| 201 | NDI₃ | CH₃CH₂CH₂CH₂N | CH₂CH₂CH₂ | CH₃ | CH₃O | 1-10 |
| 202 | NDI₃ | CH₃CH₂CH₂CH₂N | CH₂CH₂CH₂ | CH₃O | CH₃ | 10-20 |
| 203 | NDI₃ | CH₃CH₂CH₂CH₂N | CH₂CH₂CH₂ | CH₃ | CH₃O | 20-30 |
| 204 | NDI₃ | NHCONH | CH2CH₂CH₂ | CH₃O | CH₃ | 1-10 |
| 205 | NDI₃ | NHCONH | CH₂CH₂CH₂ | CH₃ | CH₃O | 10-20 |
| 206 | NDI₃ | NHCONH | CH₂CH₂CH₂ | CH₃O | CH₃ | 20-30 |
| 207 | NDI₃ | HS | CH₂CH₂CH₂ | CH₃ | CH₃O | 1-10 |
| 208 | NDI₃ | HS | CH₂CH₂CH₂ | CH₃O | CH₃ | 10-20 |
| 209 | NDI₃ | HS | CH₂CH₂CH₂ | CH₃ | CH₃O | 20-30 |
| 210 | NDI₃ | HO | CH₂CH₂CH₂ | CH₃O | CH₃ | 1-10 |
| 211 | NDI₃ | HO | CH₂CH₂CH₂ | CH₃ | CH₃O | 10-20 |
| 212 | NDI₃ | HO | CH₂CH₂CH₂ | CH₃O | CH₃ | 20-30 |
| 213 | HDI₃ | NH | CH₂CH₂CH₂ | CH3 | CH3O | 1-10 |
| 214 | HDI₃ | NH | CH₂CH₂CH₂ | CH₃O | CH₃ | 10-20 |
| 215 | HDI₃ | NH | CH₂CH₂CH₂ | CH₃ | CH₃O | 20-30 |
| 216 | HDI₃ | NHCH₂CH₂NH | CH₂CH₂CH₂ | CH₃O | CH₃ | 1-10 |
| 217 | HDI₃ | NHCH₂CH₂NH | CH₂CH₂CH₂ | CH₃ | CH₃O | 10-20 |
| 218 | HDI₃ | NHCH₂CH₂NH | CH₂CH₂CH₂ | CH₃O | CH₃ | 20-30 |
| 219 | HDI₃ | (C₆H₆)N | CH₂ | CH₃ | CH₃O | 1-10 |
| 220 | HDI₃ | (C₆H₆)N | CH₂ | CH₃O | CH₃ | 10-20 |
| 221 | HDI₃ | (C₆H₆)N | CH₂ | CH₃ | CH₃O | 20-30 |
| 222 | HDI₃ | NHCH₂CH₂NHCH₂CH₂NH | CH₂CH₂CH₂ | CH₃O | CH₃ | 1-10 |
| 223 | HDI₃ | NHCH₂CH₂NHCH₂CH₂NH | CH₂CH₂CH₂ | CH₃ | CH₃O | 10-20 |
| 224 | HDI₃ | NHCH₂CH₂NHCH₂CH₂NH | CH₂CH₂CH₂ | CH₃O | CH₃ | 20-30 |
| 225 | HDI₃ | CH₃CH₂CH₂CH₂N | CH₂CH₂CH₂ | CH₃ | CH₃O | 1-10 |
| 226 | HDI₃ | CH₃CH₂CH₂CH₂N | CH₂CH₂CH₂ | CH₃O | CH₃ | 10-20 |
| 227 | HDI₃ | CH₃CH₂CH₂CH₂N | CH₂CH₂CH₂ | CH₃ | CH₃O | 20-30 |
| 228 | HDI₃ | NHCONH | CH₂CH₂CH₂ | CH₃O | CH₃ | 1-10 |
| 229 | HDI₃ | NHCONH | CH₂CH₂CH₂ | CH₃ | CH₃O | 10-20 |
| 230 | HDI₃ | NHCONH | CH₂CH₂CH₂ | CH₃O | CH₃ | 20-30 |
| 231 | HDI₃ | HS | CH₂CH₂CH₂ | CH₃ | CH₃O | 1-10 |
| 232 | HDI₃ | HS | CH₂CH₂CH₂ | CH₃O | CH₃ | 10-20 |
| 233 | HDI₃ | HS | CH₂CH₂CH₂ | CH₃ | CH₃O | 20-30 |
| 234 | HDI₃ | OH | CH₂CH₂CH₂ | CH₃O | CH₃ | 1-10 |
| 235 | HDI₃ | OH | CH₂CH₂CH₂ | CH₃ | CH₃O | 10-20 |
| 236 | HDI₃ | OH | CH₂CH₂CH₂ | CH₃O | CH₃ | 20-30 |
| 237 | IPDI₃ | NH | CH₂CH₂CH₂ | CH₃ | CH₃O | 1-10 |
| 238 | IPDI₃ | NH | CH₂CH₂CH₂ | CH₃O | CH₃ | 10-20 |
| 239 | IPDI₃ | NH | CH₂CH₂CH₂ | CH₃ | CH₃O | 20-30 |
| 240 | IPDI₃ | NHCH₂CH₂NH | CH₂CH₂CH₂ | CH₃O | CH₃ | 1-10 |
| 241 | IPDI₃ | NHCH₂CH₂NH | CH₂CH₂CH₂ | CH₃ | CH₃O | 10-20 |
| 242 | IPDI₃ | NHCH₂CH₂NH | CH₂CH₂CH₂ | CH₃O | CH₃ | 20-30 |
| 243 | IPDI₃ | (C₆H₆)N | CH₂ | CH₃ | CH₃O | 1-10 |
| 244 | IPDI₃ | (C₆H₆)N | CH₂ | CH₃O | CH₃ | 10-20 |
| 245 | IPDI₃ | (C₆H₆)N | CH₂ | CH₃ | CH₃O | 20-30 |
| 246 | IPDI₃ | NHCH₂CH₂NHCH₂CH₂NH | CH₂CH₂CH₂ | CH₃O | CH₃ | 1-10 |
| 247 | IPDI₃ | NHCH₂CH₂NHCH₂CH₂NH | CH₂CH₂CH₂ | CH₃ | CH₃O | 10-20 |
| 248 | IPDI₃ | NHCH₂CH₂NHCH₂CH₂NH | CH₂CH₂CH₂ | CH₃O | CH₃ | 20-30 |
| 249 | IPDI₃ | CH₃CH₂CH₂CH₂N | CH₂CH₂CH₂ | CH₃ | CH₃O | 1-10 |
| 250 | IPDI₃ | CH₃CH₂CH₂CH₂N | CH₂CH₂CH₂ | CH₃O | CH₃ | 10-20 |
| 251 | IPDI₃ | CH₃CH₂CH₂CH₂N | CH₂CH₂CH₂ | CH₃ | CH₃O | 20-30 |
| 252 | IPDI₃ | NHCONH | CH₂CH₂CH₂ | CH₃O | CH₃ | 1-10 |
| 253 | IPDI₃ | NHCONH | CH₂CH₂CH₂ | CH₃ | CH₃O | 10-20 |
| 254 | IPDI₃ IPDI₃ | NHCONH | CH₂CH₂CH₂ | CH₃O | CH₃ | 20-30 |
| 255 | IPDI₃ | HS | CH₂CH₂CH₂ | CH₃ | CH₃O | 1-10 |
| 256 | IPDI₃ | HS | CH₂CH₂CH₂ | CH₃O | CH₃ | 10-20 |
| 257 | IPDI₃ | HS | CH₂CH₂CH₂ | CH₃ | CH₃O | 20-30 |
| 258 | IPDI₃ | OH | CH₂CH₂CH₂ | CH₃O | CH₃ | 1-10 |
| 259 | IPDI₃ | OH | CH₂CH₂CH₂ | CH₃ | CH₃O | 10-20 |
| 260 | IPDI₃ | OH | CH₂CH₂CH₂ | CH₃O | CH₃ | 20-30 |
| 261 | TMCDI₃ | NH | CH₂cH₂CH₂ | CH₃ | CH₃O | 1-10 |
| 262 | TMCDI₃ | NH | CH₂CH₂CH₂ | CH₃O | CH₃ | 10-20 |
| 263 | TMCDI₃ | NH | CH₂CH₂CH₂ | CH₃ | CH₃O | 20-30 |
| 264 | TMCDI₃ | NHCH₂CH₂NH | CH₂CH₂CH₂ | CH₃O | CH₃ | 1-10 |
| 265 | TMCDI₃ | NHCH₂CH₂NH | CH₂CH₂CH₂ | CH₃ | CH₃O | 10-20 |
| 266 | TMCDI₃ | NHCH₂CH₂NH | CH₂CH₂CH₂ | CH₃O | CH₃ | 20-30 |
| 267 | TMCDI₃ | (C₆H₆)N | CH₂ | CH₃ | CH₃O | 1-10 |
| 268 | TMCDI₃ | (C₆H₆)N | CH₂ | CH₃O | CH₃ | 10-20 |
| 269 | TMCDI₃ | (C₆H₆)N | CH₂ | CH₃ | CH₃O | 20-30 |
| 270 | TMCDI₃ | NHCH₂CH₂NHCH₂CH₂NH | CH₂CH₂CH₂ | CH₃O | CH₃ | 1-10 |
| 271 | TMCDI₃ | NHCH₂CH₂NHCH₂CH₂NH | CH₂CH₂CH₂ | CH₃ | CH₃O | 10-20 |
| 272 | TMCDI₃ | NHCH₂CH₂NHCH₂CH₂NH | CH₂CH₂CH₂ | CH₃O | CH₃ | 20-30 |
| 273 | TMCDI₃ | CH₃CH₂CH₂CH₂N | CH₂CH₂CH₂ | CH₃ | CH₃O | 1-10 |
| 274 | TMCDI₃ | CH₃CH₂CH₂CH₂N | CH₂CH₂CH₂ | CH₃O | CH₃ | 10-20 |
| 275 | TMCDI₃ | CH₃CH₂CH₂CH₂N | CH₂CH₂CH₂ | CH₃ | CH₃O | 20-30 |
| 276 | TMCDI₃ | NHCONH | CH₂CH₂CH₂ | CH₃O | CH₃ | 1-10 |
| 277 | TMCDI₃ | NHCONH | CH₂CH₂CH₂ | CH₃ | CH₃O | 10-20 |
| 278 | TMCDI₃ | NHCONH | CH₂CH₂CH₂ | CH₃O | CH₃ | 20-30 |
| 279 | TMCDI₃ | HS | CH₂CH₂CH₂ | CH₃ | CH₃O | 1-10 |
| 280 | TMCDI₃ | HS | CH₂CH₂CH₂ | CH₃O | CH₃ | 10-20 |
| 281 | TMCDI₃ | HS | CH₂CH₂CH₂ | CH₃ | CH₃O | 20-30 |
| 282 | TMCDI₃ | HO | CH₂CH₂CH₂ | CH₃O | CH₃ | 1-10 |
| 283 | TMCDI₃ | HO | CH₂CH₂CH₂ | CH₃ | CH₃O | 10-20 |
| 284 | TMCDI₃ | HO | CH₂CH₂CH₂ | CH₃O | CH₃ | 20-30 |
| 285 | HMDI₃ | NH | CH₂CH₂CH₂ | CH₃ | CH₃O | 1-10 |
| 286 | HMDI₃ | NH | CH₂CH₂CH₂ | CH₃O | CH₃ | 10-20 |
| 287 | HMDI₃ | NH | CH₂CH₂CH₂ | CH₃ | CH₃O | 20-30 |
| 288 | HMDI₃ | NHCH₂CH₂NH | CH₂CH₂CH₂ | CH₃O | CH₃ | 1-10 |
| 289 | HMDI₃ | NHCH₂CH₂NH | CH₂CH₂CH₂ | CH₃ | CH₃O | 10-20 |
| 290 | HMDI₃ | NHCH₂CH₂NH | CH₂CH₂CH₂ | CH₃O | CH₃ | 20-30 |
| 291 | HMDI₃ | (C₆H₆)N | CH₂ | CH₃ | CH₃O | 1-10 |
| 292 | HMDI₃ | (C₆H₆)N | CH₂ | CH₃O | CH₃ | 10-20 |
| 293 | HMDI₃ | (C₆H₆)N | CH₂ | CH₃ | CH₃O | 20-30 |
| 294 | HMDI₃ | NHCH₂CH₂NHCH₂CH₂NH | CH₂CH₂CH₂ | CH₃O | CH₃ | 1-10 |
| 295 | HMDI₃ | NHCH₂CH₂NHCH₂CH₂NH | CH₂CH₂CH₂ | CH₃ | CH₃O | 10-20 |
| 296 | HMDI₃ | NHCH₂CH₂NHCH₂CH₂NH | CH₂CH₂CH₂ | CH₃O | CH₃ | 20-30 |
| 297 | HMDI₃ | CH₃CH₂CH₂CH₂N | CH₂CH₂CH₂ | CH₃ | CH₃O | 1-10 |
| 298 | HMDI₃ | CH₃CH₂CH₂CH₂N | CH₂CH₂CH₂ | CH₃O | CH₃ | 10-20 |
| 299 | HMDI₃ | CH₃CH₂CH₂CH₂N | CH₂CH₂CH₂ | CH₃ | CH₃O | 20-30 |
| 300 | HMDI₃ | NHCONH | CH₂CH₂CH₂ | CH₃O | CH₃ | 1-10 |
| 301 | HMDI₃ | NHCONH | CH₂CH₂CH₂ | CH₃ | CH₃O | 10-20 |
| 302 | HMDI₃ | NHCONH | CH₂CH₂CH₂ | CH₃O | CH₃ | 20-30 |
| 303 | HMDI₃ | HS | CH₂CH₂CH₂ | CH₃ | CH₃O | 1-10 |
| 304 | HMDI₃ | HS | CH₂CH₂CH₂ | CH₃O | CH₃ | 10-20 |
| 305 | HMDI₃ | HS | CH₂CH₂CH₂ | CH₃ | CH₃O | 20-30 |
| 306 | HMDI₃ | HO | CH₂CH₂CH₂ | CH₃O | CH₃ | 1-10 |
| 307 | HMDI₃ | HO | CH₂CH₂CH₂ | CH₃ | CH₃O | 10-20 |
| 308 | HMDI₃ | HO | CH₂CH₂CH₂ | CH₃O | CH₃ | 20-30 |

### Example 13

To a four necked flask (500 mL) equipped with a thermometer, a reflux condenser, a stirrer, and a funnel, 128 g of the compound 89, 46.5 g of polycarbonate diol (Mn=1000), 6 g of polyethyleneglycol adipate (Mn=2000), 6 g of dimethyopropionic acid, 14 g of N-methyl pyrrolidone, 0.4 g of DBTDL, and 10 g of acetone were separately added. The mixture was allowed to react at 70°C until the amount of free isocyanate was less than 0.5 weight%, and thereby a polyurethane prepolymer was obtained. The prepolymer was cooled to 40°C, and then 6 g of triethylamine was added dropwise for neutralizing, 120 g of deionized water added for shearing, and 2 g of ethyldiamine added for chain extension. Finally, acetone was removed under reduced pressure distillation to yield a polyurethane emulsion with solid content of 42 weight%.

### Example 14

To a four necked flask (500 mL) equipped with a thermometer, a reflux condenser, a stirrer, and a funnel, 32 g of IPDI, 46.5 g of polycarbonate diol (Mn=1000), 6 g of polyethyleneglycol adipate (Mn=2000), 6 g of dimethyopropionic acid, 14 g of N-methyl pyrrolidone, 0.4 g of DBTDL, and 10 g of acetone were separately added. The mixture was allowed to react at 70°C until the amount of free isocyanate was less than 0.5 weight%, and thereby a polyurethane prepolymer was obtained. The prepolymer was cooled to 40°C, and then 6 g of triethylamine was added dropwise for neutralizing, 120 g of deionized water added for shearing, and 2 g of ethyldiamine added for chain extension. Finally, acetone was removed under reduced pressure distillation to yield a polyurethane emulsion with solid content of 40 weight%.

### Example 15

### Performance measurement

To evaluate the effectiveness of polyurethane (PU), epoxy resin, polymethacrylate (PMA), polyacrylamide(PAM), or phenolic resingloss prepared by isocyanate trimers modified with silane or functional polysiloxane, the gross, contact angle, surface drying time (h), adhesion, hardness, and flexibility of a coating of waterborne polyurethane prepared by isocyanate trimers modified with silane or functional polysiloxane were measured, and part of results were listed in Table 3.

**Table 3 Performance index of waterborne polyurethane prepared by isocyanate trimer modified with silane or functional polysiloxane**

| No. | Si-compound | Gloss | Contact angle(θ) | Surface drying time (min, 25°C) | Hardness | Adhesion | Flexibility (mm) |
|---|---|---|---|---|---|---|---|
| 309(b) | - | 82 | 55 | 60 | 0.4 | 3 | 4 |
| 310 | 81 | 90 | 72 | 25 | 0.6 | 1 | 2 |
| 311 | 82 | 90 | 72 | 24 | 0.6 | 1 | 2 |
| 312 | 83 | 90 | 72 | 24 | 0.6 | 1 | 2 |
| 313 | 84 | 92 | 72 | 24 | 0.6 | 1 | 2 |
| 314 | 85 | 91 | 70 | 24 | 0.6 | 2 | 2 |
| 315 | 86 | 90 | 70 | 23 | 0.6 | 2 | 2 |
| 316 | 87 | 90 | 71 | 22 | 0.6 | 1 | 2 |
| 317 | 88 | 90 | 71 | 23 | 0.6 | 1 | 2 |
| 318 | 89 | 90 | 71 | 24 | 0.6 | 1 | 2 |
| 319 | 90 | 91 | 72 | 20 | 0.6 | 1 | 2 |
| 320 | 91 | 93 | 72 | 20 | 0.6 | 1 | 2 |
| 321 | 92 | 93 | 72 | 19 | 0.6 | 1 | 2 |
| 322 | 93 | 94 | 72 | 19 | 0.6 | 1 | 2 |
| 323 | 94 | 94 | 72 | 19 | 0.6 | 1 | 2 |
| 324 | 95 | 90 | 72 | 22 | 0.6 | 1 | 2 |
| 325 | 96 | 90 | 72 | 22 | 0.6 | 1 | 2 |
| 326 | 97 | 90 | 72 | 22 | 0.6 | 1 | 2 |
| 327 | 98 | 90 | 72 | 24 | 0.6 | 1 | 2 |
| 328 | 99 | 90 | 71 | 25 | 0.6 | 2 | 2 |
| 329 | 100 | 90 | 71 | 25 | 0.6 | 2 | 2 |
| 330 | 237 | 98 | 77 | 21 | 0.7 | 1 | 1 |
| 331 | 238 | 97 | 77 | 21 | 0.7 | 0 | 1 |
| 332 | 239 | 98 | 77 | 22 | 0.7 | 0 | 1 |
| 333 | 240 | 99 | 77 | 19 | 0.7 | 1 | 1 |
| 334 | 241 | 98 | 77 | 19 | 0.7 | 1 | 1 |
| 335 | 242 | 98 | 78 | 19 | 0.7 | 0 | 1 |
| 336 | 243 | 97 | 78 | 20 | 0.7 | 1 | 1 |
| 337 | 244 | 98 | 78 | 20 | 0.7 | 1 | 1 |
| 338 | 245 | 99 | 78 | 20 | 0.7 | 1 | 1 |
| 339 | 246 | 98 | 78 | 20 | 0.7 | 1 | 1 |
| 340 | 247 | 97 | 78 | 20 | 0.7 | 0 | 1 |
| 341 | 248 | 99 | 78 | 20 | 0.7 | 0 | 1 |
| 342 | 249 | 97 | 78 | 19 | 0.7 | 1 | 1 |
| 343 | 250 | 97 | 78 | 21 | 0.7 | 0 | 1 |
| 344 | 251 | 97 | 78 | 21 | 0.7 | 0 | 1 |
| 345 | 252 | 98 | 78 | 21 | 0.7 | 1 | 1 |
| 346 | 253 | 98 | 78 | 22 | 0.7 | 0 | 1 |
| 347 | 254 | 98 | 77 | 22 | 0.7 | 0 | 1 |
| 348 | 255 | 99 | 77 | 21 | 0.7 | 1 | 1 |
| 349 | 256 | 98 | 78 | 21 | 0.7 | 0 | 1 |
| 350 | 257 | 99 | 77 | 21 | 0.7 | 0 | 1 |
| 351 | 258 | 98 | 77 | 21 | 0.7 | 1 | 1 |
| 352 | 259 | 99 | 77 | 22 | 0.7 | 0 | 1 |

The invention provides an isocyanate trimer modified with silane or functional polysiloxane and a method preparing the same. Waterborne polyurethane prepared by the modified isocyanate trimer has higher gloss, larger contact angle, greater hardness, drying faster, better adhesion, and better flexibility. Any modified isocyanurate and preparation method above, and the similar, will fall in the range of this invention.

## Claims

1. An isocyanate trimer modified with silane or functional linear polysiloxane, having a formula of 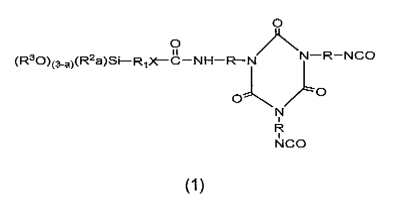 or 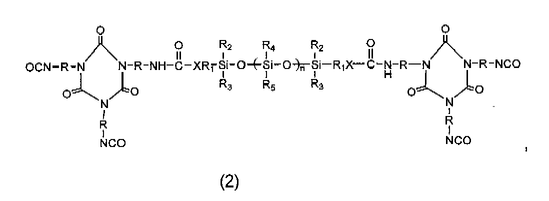 wherein
R represents tolyl, ethylphenyl, diphenylmethyl, 1,5-naphthyl, hexamethylene, isophorone, 2,2,6-trimethylcyclohexyl, or 4,4-dicyclohexylmethyl;
R₁ represents saturated or unsaturated and linear or branched C₁-₈ alkyl, cycloalkyl, aryl, or a homologue of aromatic hydrocarbon;
R² and R³ at each occurrence separately represent C₁₋₆ alkyl, aryl, trialkylsilyl, or methoxyalkoxyl;
a is an integer from 0 to 3;
R₁, R₂, R₃, R₄, and R₅ at each occurrence separately represent saturated or unsaturated and linear or branched C₁₋₁₂ alkyl, cycloalkyl, alkoxyl, or aryl;
X represents -NH-, -NHCH₂CH₂NH-,-NHCH₂CH₂NHCH₂CH₂NH-, -N-, -NHCONH-, 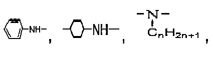 -S-, or -O-; and
n is an integer which is ≥ 0.

2. A method of preparing the isocyanate trimer represented by formula (1) or (2) of claim 1, comprising steps of
a) dissolving an isocyanate trimer into an inert solvent;
b) adding dropwise to a mixture of silane or functional polysiloxane and a catalyst;
c) stirring the resultant solution at room temperature and in a dry surrounding; and -
d)removing the inert solvent under reduced pressure distillation to yield an isocyanate trimer modified with silane or functional polysiloxane.

3. The method of claim 2, wherein the isocyanate trimer is represented by a formula of 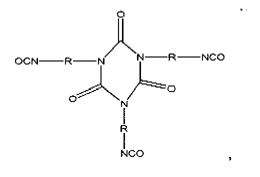 wherein R represents tolyl, ethylphenyl, diphenylmethyl, 1,5-naphthyl, hexamethylene, isophorone, 2,2,6-trimethylcyclohexyl, or 4,4-dicyclohexylmethyl, and an isocyanate is toluene diisocyanate, ethylbenzene diisocyanate, methylene diphenyl diisocyanate, 1,5-naphthalene diisocyanate, hexamethylene diisocyanate, isophorone diisocyanate, 1,4-diisocyanate-2,2,6-trimethyl cyclohexane, or 4,4-bis(isocyanatecyclohexyl)methane.

4. The method of claim 2 or 3, where the silane or functional polysiloxane is represented by formula of
(R³O)₍₃₋ₐ₎(R²a)Si-R₁XH
or 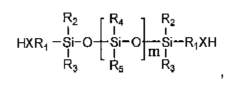 wherein
R² and R³ at each occurrence separately represent C₁₋₆ alkyl, aryl, trialkylsilyl, or methoxylalkoxyl;
a is an integer from 0 to 3;
R₁, R₂ R₃, R₄, and R₅ at each occurrence separately represent saturated or unsaturated and linear or branched C₁₋₁₂ alkyl, aryl, cycloalkyl, alkoxyl, or cycloalkoxyl;
X represents -NH-, -NHCH₂CH₂NH-, -NHCH₂CH₂NHCH₂CH₂NH-, N-,
-NHCONH-, 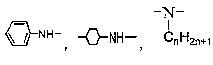 wherein n ≥0, -S-, or -O-; and
m is an integer from 0 to 30.

5. The method of claim 2 or 3, wherein the catalyst is an organic selected form dibutyltin dilaurate, dibutyltin maleate, dibutyltin diacetate, dibutyltin dilaurylmercaptan, and dimethyltin dichloride.

6. The method of claim 5, wherein the amount of the organic tin compound used is between 0.01 and 0.5 weight% of the total amount of the isocyanate trimer and silicon compound.

7. The method of claim 2 or 3, wherein the inert organic solvent which does not react with the reactants is hydrocarbon, chlorinated hydrocarbon, ester, or ether.

8. The method of claim 7, wherein the hydrocarbon solvent is petroleum ether, linear or branched hydrocarbon with low boiling point, benzene, toluene, or xylene, the chlorinated hydrocarbon being chloroform, chlorobenzene, dichloromethane, or dichloroethane, the ester being ethyl acetate, and the ether being aether or tetrahydrofuran.

9. The method of claim 2 or 3, wherein a molar ratio of the isocyanate trimer to the active hydrogen of silanes or polysiloxane is 1:1.

## Amended claims

### Amended claims under Art. 19.1 PCT

**1.** An isocyanate trimer modified with silane or functional linear polysiloxane, having a formula of 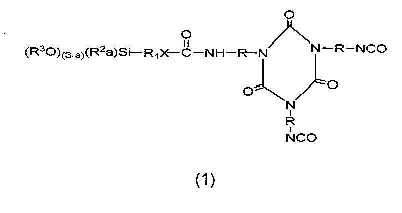 or 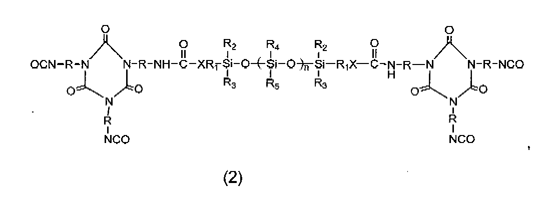 wherein
R represents tolyl, ethylphenyl, diphenylmethyl, 1,5-naphthyl, hexamethylene, isophorone, 2,2,6-trimethylcyclohexyl, or 4,4-dicyclohexylmethyl;
R₁ represents saturated or unsaturated and linear or branched C₁₋₈ alkyl, cycloalkyl, aryl, or a homologue of aromatic hydrocarbon;
R² and R³ at each occurrence separately represent C₁₋₆ alkyl, aryl, trialkylsilyl, or methoxyalkoxyl;
a is an integer from 0 to 3;
R₁, R₂, R₃, R₄, and R₅ at each occurrence separately represent saturated or unsaturated and linear or branched C₁₋₁₂ alkyl, cycloalkyl, alkoxyl, or aryl;
X represents -NH-, -NHCH₂CH₂NH-,-NHCH₂CH₂NHCH₂CH₂NH-, -N-,
-NHCONH-, 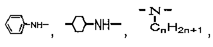 -S-, or-O-; and
n is an integer which ins ≥ 0.

**2.** A method of preparing the isocyanate trimer represented by formula (1) or (2) of claim 1, comprising steps of
a)dissolving an isocyanate trimer into an inert solvent;
b) adding dropwise to a mixture of silane or functional polysiloxane and a catalyst;
c) stirring the resultant solution at room temperature and in a dry surrounding; and
d) removing the inert solvent under reduced pressure distillation to yield an isocyanate trimer modified with silane or functional polysiloxane.

**3.** The method of claim 2, wherein the isocyanate trimer is represented by a formula of 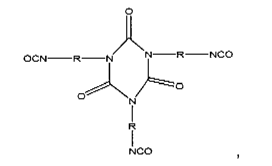 wherein R represents tolyl, ethylphenyl, diphenylmethyl, 1,5-naphthyl, hexamethylene, isophorone, 2,2,6-trimethylcyclohexyl, or4,4-dicyclohexylmethyl, and an isocyanate is toluene diisocyanate, ethylbenzene diisocyanate, methylene diphenyl diisocyanate, 1,5-naphthalene diisocyanate, hexamethylene diisocyanate, isophorone diisocyanate, 1,4-diisocyanate-2,2,6-trimethyl cyclohexane, or 4,4-bis(isocyanatecyclohexyl)methane.

**4.** The method of claim 2 or 3, where the silane or functional polysiloxane is represented by formula of
(R³O)₍₃₋ₐ₎(R²a)Si-R₁XH
or 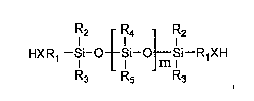 wherein
R² and R³ at each occurrence separately represent C₁₋₆ alkyl, aryl, trialkylsilyl, or methoxylalkoxyl;
a is an integer from 0 to 3;
R₁, R₂, R₃, R₄, and R₅ at each occurrence separately represent saturated or unsaturated and linear or branched C₁₋₁₂ alkyl, aryl, cycloalkyl, alkoxyl, or cycloalkoxyl;
X represents -NH-, -NHCH₂CH₂NH-, -NHCH₂CH₂NHCH₂CH₂NH-, N-,
-NHCONH-, 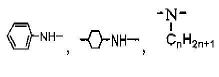 wherein n ≥0, -S-, or -O-; and
m is an integer from 0 to 30.

**5.** The method of claim 2 or 3, wherein the catalyst is an organic selected form dibutyltin dilaurate, dibutyltin maleate, dibutyltin diacetate, dibutyltin dilaurylmercaptan, and dimethyltin dichloride.

**6.** The method of claim 5, wherein the amount of the organic tin compound used is between 0.01 and 0.5 weight% of the total amount of the isocyanate trimer and silicon compound.

**7.** The method of claim 2 or 3, wherein the inert organic solvent which does not react with the reactants is hydrocarbon, chlorinated hydrocarbon, ester, or ether.

**8.** The method of claim 7, wherein the hydrocarbon solvent is petroleum ether, linear or branched hydrocarbon with low boiling point, benzene, toluene, or xylene, the chlorinated hydrocarbon being chloroform, chlorobenzene, or dichloromethane.

**9.** The method of claim 7, wherein the ester is ethyl acetate and the ether is aether or tetrahydrofuran.

**10.** The method of claim 2 or 3, wherein a molar ratio of the isocyanate trimer to the active hydrogen of silanes or polysiloxane is 1:1.
